(19) Europäisches Patentamt
European Patent Office
Office européen des brevets

(11) **EP 0 634 384 B1**

(12) **EUROPÄISCHE PATENTSCHRIFT**

(45) Veröffentlichungstag und Bekanntmachung des Hinweises auf die Patenterteilung:
**14.01.1998 Patentblatt 1998/03**

(51) Int. Cl.$^6$: **C07C 17/087**, C07C 19/08

(21) Anmeldenummer: **94110536.3**

(22) Anmeldetag: **06.07.1994**

(54) **Herstellung von 2-H-Heptafluorpropan**

Preparation of 2-H-heptafluoropropane

Préparation de 2-H-heptafluoropropane

(84) Benannte Vertragsstaaten:
**BE CH DE DK ES FR GB GR IT LI NL PT SE**

(30) Priorität: **14.07.1993 DE 4323054**

(43) Veröffentlichungstag der Anmeldung:
**18.01.1995 Patentblatt 1995/03**

(73) Patentinhaber:
**SOLVAY (Société Anonyme)**
**B-1050 Bruxelles (BE)**

(72) Erfinder:
• **Franz, Raimund, Dr.**
**D-65779 Kelkheim (DE)**

• **Siegemund, Günter, Dr.**
**D-65719 Hofheim (DE)**

(74) Vertreter:
**Jacques, Philippe et al**
**Solvay S.A.**
**Département Propriété Industrielle**
**310, rue de Ransbeek**
**1120 Bruxelles (BE)**

(56) Entgegenhaltungen:
**BE-A- 652 779      GB-A- 902 590**

**Beschreibung**

Gegenstand der Erfindung ist ein neues Verfahren zur Herstellung von 2-H-Heptafluorpropan. 2-H-Heptafluorpropan (R 227) ist ein chlorfreier, und somit für die stratosphärische Ozonschicht unschädlicher, bei ca. -18°C siedender Fluorkohlenwasserstoff von wachsender technischer Bedeutung, z. B. als Treibmittel. Seit seiner erstmaligen Herstellung im Jahr 1946 durch Hydrolyse einer Grignard-Verbindung sind einige weitere Zugangswege bekannt geworden. Für eine Umsetzung in den technischen Maßstab kommen überwiegend solche Verfahren in Betracht, die in einer direkten oder indirekten Addition von Fluorwasserstoff an Hexafluorpropen bestehen:

$$CF_3\text{-}CF=CF_2 + HF \rightarrow CF_3\text{-}CHF\text{-}CF_3$$

Die älteste Methode dieser Art ist die von W. T. Miller et al. veröffentlichte Umsetzung von Hexafluorpropen mit Kaliumfluorid in Formamid (JACS 82, 3091 - 3099 (1960)):

$$CF_3\text{-}CF=CF_2 \xrightarrow{\text{HCONH}_2/\text{KF}} CF_3\text{-}CHF\text{-}CF_3 \text{ Umsatz: 60 \%}$$

Als Nachteil fällt bei diesem Verfahren ins Gewicht, daß das zu dieser indirekten HF-Addition nötige Wasserstoff-Atom dem Reaktionsmedium (d.h. dem Formamid) durch Abspaltung entnommen wird, was zur Bildung unerwünschter Nebenprodukte führt. Daher wurde später angestrebt, die Addition durch direkte Einwirkung von Fluorwasserstoff auf Hexafluorpropen, zweckmäßig in Gegenwart eines Katalysators, zu erreichen. Diese Versuche gelangen jedoch bis jetzt nur unter Anwendung hoher Reaktionstemperaturen. Nach einem in der GB-PS 902590 beschriebenen Verfahren setzt sich ein äquimolares, gasförmiges Gemisch aus Hexafluorpropen und Fluorwasserstoff, das einen mit Aktivkohle gefüllten Rohrreaktor durchströmt, erst bei Temperaturen zwischen 250 und 450°C zu 2-H-Heptafluorpropan um.

Das Arbeiten mit Gasgemischen bei so hohen Temperaturen ist stets mit Problemen technischer und chemischer Art verbunden. Es werden hohe Anforderungen an die Arbeitssicherheit und an die Korrosionsfestigkeit der betroffenen Werkstoffe gestellt; daneben ist bekannt, daß bei Fluorkohlenwasserstoffen oberhalb von 250°C mit einem zunehmenden thermischen Abbau zu rechnen ist, der ab 500°C erhebliche Ausmaße annimmt. In derselben Apparatur, wie sie für die Herstellung von 2-H-Heptafluorpropan benötigt wird, erfolgt nach GB-PS 905 617 bei 545°C eine thermische Reaktion des 2-H-Heptafluorpropans zu Perfluorpropan (17 % d. Th.) und 2-H-Nonafluorisobutan (27 % d. Th.). Somit ist auch schon in dem für die Herstellung des 2-H-Heptafluorpropans oben genannten Temperaturbereich mit dem Auftreten von Spuren dieser Produkte zu rechnen. Hierbei muß beachtet werden, daß aus 2-H-Nonafluorisobutan das hochtoxische Perfluorisobuten bei erhöhter Temperatur leicht entstehen kann.

Es wurde nun überraschenderweise gefunden, daß Hexafluorpropen und Fluorwasserstoff außerordentlich leicht im Sinne einer Addition miteinander reagieren, wenn als fester Katalysator ein schwachbasischer Ionenaustauscher verwendet wird, dessen reaktive Zentren aus tertiären Aminogruppen bestehen. Die Umsetzung bedarf keiner Wärmezufuhr; vielmehr ist die Reaktion exotherm.

Gegenstand der vorliegenden Erfindung ist somit ein Verfahren zur Herstellung von 2-H-Heptafluorpropan aus Hexafluorpropen, dadurch gekennzeichnet, daß man Hexafluorpropen mit Fluorwasserstoff in Gegenwart eines schwachbasischen Ionenaustauschers, dessen reaktive Zentren aus tertiären Aminogruppen bestehen, umsetzt.

Als Reaktionsapparate für die erfindungsgemäße Umsetzung können geschlossene Druckgefäße dienen, in welche die Reaktanden mit Hilfe von Pumpen eingebracht werden; es ist jedoch auch eine drucklose, kontinuierliche Reaktionsführung möglich, für die sich einfache Rohrreaktoren oder auch Wirbelschicht-Reaktoren besonders gut eignen. Es ist dabei zweckmäßig, den zu verwendenden Reaktor aus einem Material zu fertigen, das gegen Fluorwasserstoff resistent ist (z. B. rostfreier Stahl, Nickel, ®Hastelloy oder Kunststoff).

Als schwachbasische Ionenaustauscher, deren reaktive Zentren aus tertiären Aminogruppen bestehen, können handelsübliche Materialien eingesetzt werden, wie sie zur Wasseraufbereitung oder zu vergleichbaren Zwecken verwendet werden, z. B. das Produkt ®Amberlite IRA 93 SP der Fa. Rohm & Haas. Der Ionenaustauscher wird als rieselfähige Schüttung oder als Suspension in einer hochsiedenden Inertflüssigkeit, wie Paraffinöl, in den Reaktor eingeführt. Es ist hierbei zweckmäßig, zuvor etwa enthaltenes Wasser, soweit ohne besonderen Aufwand möglich, zu entfernen, z. B. durch Trocknung bei leicht erhöhter Temperatur im Vakuum bis zur Gewichtskonstanz.

Je nach Bauart der Apparatur kann es von Vorteil sein, die stark exotherme Umsetzung durch eine Vor-Beladung des einzusetzenden Ionenaustauschers mit Fluorwasserstoff zu mäßigen. Diese Vor-Beladung kann in der Reaktionsapparatur selbst oder auch extern erfolgen und sollte maximal 33 g, bevorzugt 8 - 25 g, insbesondere 18 - 23 g Fluorwasserstoff je 100 g des Ionenaustauschers betragen.

Die Reaktanden der erfindungsgemäßen Umsetzung, d. h. Hexafluorpropen und Fluorwasserstoff, werden zweckmäßig gasförmig und simultan im molaren Verhältnis 0,9 - 1,1 : 1, vorzugsweise 1 : 1, d. h. im Gewichtsverhältnis 7,5 : 1, in den Reaktor eingeführt, der den gewählten Ionenaustauscher enthält. Die Isolierung des 2-H-Heptafluorpropans erfolgt im allgemeinen durch einfaches Kondensieren des Gases, welches man beim Entspannen des verwendeten Druckgefäßes oder als Produkt einer kontinuierlich arbeitenden Apparatur erhält. Es kann jedoch notwendig sein, das Rohprodukt durch alkalische bzw. saure Waschvorgänge von mitgerissenen Spuren an Fluorwasserstoff oder Aminen zu befreien. Auch eine destillative Aufarbeitung kann bei hohen Reinheitsanforderungen notwendig sein.

Die Reaktionstemperaturen für die erfindungsgemäße Umsetzung richten sich im wesentlichen nach der thermischen Belastbarkeit des verwendeten Ionenaustauschers. Sie liegen im allgemeinen bei 20 - 120°C, bevorzugt bei 30 - 90°C, insbesondere bei 50 - 70°C.

Die folgenden Ausführungsbeispiele dienen zur Illustration des erfindungsgemäßen Verfahrens.

Beispiel 1

120 g des Ionenaustauschers ®Amberlite (Fa. Rohm & Haas) Typ IRA 93 SP (zuvor bis zur Gewichtskonstanz bei 80°C im Vakuum getrocknet) wurden in einem verschlossenen Kunststoff-Gefäß unter Schütteln mit 14 g Fluorwasserstoffgas behandelt; der Fluorwasserstoff wurde hierbei gleichmäßig und vollständig absorbiert. Ein zwecks Außenkühlung ummanteltes, senkrecht montiertes Rohr aus rostfreiem Stahl von 60 cm Länge und 3 cm Innendurchmesser wurde als Reaktor eingesetzt. Das Rohr war mit einem feinmaschigen Siebboden versehen. Ein zentrisch eingebautes, am unteren Ende verschlossenes, dünnes Metallrohr diente zur Aufnahme von Thermoelementen. Der Reaktor wurde mit dem mit HF vorbeladenen Ionenaustauscher gefüllt, verschlossen und mit einer Vorrichtung zur Entnahme von Gasproben sowie mit einer Kühlfalle verbunden. Unterhalb des Siebbodens wurde nunmehr Hexafluorpropen mit einer Geschwindigkeit von 180 mMol/h eingeleitet, und nach Anspringen der Reaktion (erkennbar am Temperaturanstieg am Fuß des Rohres) Fluorwasserstoff mit einer Geschwindigkeit von ebenfalls 180 mMol/h zudosiert. Die Innentemperatur stieg dabei auf ca. 60°C; bei diesem Wert wurde die Reaktionswärme durch einen Kühlwasserfluß von 23°C abgeführt. 50 Minuten nach Beginn der Umsetzung wurde die erste Gasprobe entnommen, alle weiteren Proben in halbstündigen Abständen. Es wurde ein Gehalt von 94,6 % 2-H-Heptafluorpropan im Rohgasgemisch erreicht (GC-Bedingungen: 5 m - ®Porasil-C-Säule, 5 % OPN (Oxy-dipropionitril), 80°C isotherm, Wärmeleitfähigkeitsdetektor).

Beispiel 2

In einem Rührautoklaven aus ®Hastelloy-C von 300 ml Fassungsvermögen wurden 15 g ®Amberlite IRA 93 SP, vor-beladen mit 2,8 g Fluorwasserstoff, gegeben und in 100 g eines bei 216°C siedenden Perfluor-polyethers suspendiert. Nach dem Verschließen des Autoklaven wurden 14 g Hexafluorpropen eingepreßt und das Gemisch 6,5 h bei 50°C unter Eigendruck gerührt. Das Gaschromatogramm (Bedingungen wie in Beispiel 1) einer danach entnommenen Probe zeigte einen Gehalt von 85,5 % 2-H-Heptafluorpropan und ca. 14,1 % Hexafluorpropen im Rohprodukt an.

**Patentansprüche**

1. Verfahren zur Herstellung von 2-H-Heptafluorpropan aus Hexafluorpropen, dadurch gekennzeichnet, daß man Hexafluorpropen mit Fluorwasserstoff in Gegenwart eines schwachbasischen Ionenaustauschers, dessen reaktive Zentren aus tertiären Aminogruppen bestehen, umsetzt.

2. Verfahren nach Anspruch 1, dadurch gekennzeichnet, daß man Hexafluorpropen und HF im molaren Verhältnis von 0,9:1 bis 1,1:1 einsetzt.

3. Verfahren nach Anspruch 1 oder 2, dadurch gekennzeichnet, daß man die Umsetzung bei einer Temperatur von 20 bis 120°C durchführt.

4. Verfahren nach Anspruch 1 oder 2, dadurch gekennzeichnet, daß man die Umsetzung bei einer Temperatur von 30 bis 90°C durchführt.

5. Verfahren nach Anspruch 1 oder 2, dadurch gekennzeichnet, daß man die Umsetzung bei einer Temperatur von 50 bis 70°C durchführt.

**Claims**

1. A process for the preparation of 2-H-heptafluoropropane from hexafluoropropene, characterized in that hexafluoropropene is reacted with hydrogen fluoride in the presence of a weakly basic ion exchanger whose reactive centers comprise tertiary amino groups.

2. The process as claimed in claim 1, characterized in that hexafluoropropene and HF are used in a molar ratio of 0.9:1 to 1.1:1.

3. The process as claimed in claim 1 or 2, characterized in that the reaction is carried out at a temperature of 20 to 120°C.

4. The process as claimed in claim 1 or 2, characterized in that the reaction is carried out at a temperature of 30 to 90°C.

5. The process as claimed in claim 1 or 2, characterized in that the reaction is carried out at a temperature of 50 to 70°C.

**Revendications**

1. Procédé de préparation de 2-H-heptafluoropropane à partir d'hexafluoropropène, caractérisé en ce qu'on fait réagir de l'hexafluoropropène avec du fluorure d'hydrogène en présence d'un échangeur d'ions faiblement basique, dont les centres réactionnels sont constitués de groupes amino tertiaires.

2. Procédé selon la revendication 1, caractérisé en ce qu'on utilise l'hexafluoropropène et le HF en un rapport molaire de 0,9 : 1 à 1,1 : 1.

3. Procédé selon la revendication 1 ou 2, caractérisé en ce qu'on effectue la réaction à une température de 20 à 120°C.

4. Procédé selon la revendication 1 ou 2, caractérisé en ce qu'on effectue la réaction à une température de 30 à 90°C.

5. Procédé selon la revendication 1 ou 2, caractérisé en ce qu'on effectue la réaction à une température de 50 à 70°C.